# EUROPEAN PATENT SPECIFICATION

(11) **EP 3 661 418 B1**
(45) Date of publication and mention of the grant of the patent: **01.09.2021**
(21) Application number: 18729903.7
(22) Date of filing: 30.05.2018
(51) Int. Cl.: A61B 5/12, A61B 5/00

(54) **METHOD AND APPARATUS FOR MEASURING THE ACOUSTIC REFLEX WITH ARTIFACT MANAGEMENT BY USING MULTIPLE PROBE TONES**
VERFAHREN UND VORRICHTUNG ZUR MESSUNG DES AKUSTISCHEN REFLEXES MIT ARTEFAKTBEHANDLUNG UNTER VERWENDUNG MEHRERER ABTASTTÖNE
PROCÉDÉ ET APPAREIL POUR MESURER LE RÉFLEXE ACOUSTIQUE AVEC UNE GESTION D'ARTEFACTS EN UTILISANT DE MULTIPLES TONALITÉS DE SONDE

(30) Priority: 01.08.2017 US 201715665533
(43) Date of publication of application: 10.06.2020
(73) Proprietor: Path Medical GmbH, 82110 Germering (DE)
(72) Inventor: LODWIG, Andre, 82237 Wörthsee (DE); KANDZIA, Florian, 81243 München (DE)
(74) Representative: Zimmermann & Partner Patentanwälte mbB
(86) International application number: PCT/EP2018/064249
(87) International publication number: WO 2019/025053

(56) References cited:
- FR-A1- 2 971 931
- US-A- 5 885 225
- US-A1- 2012 302 859
- US-A1- 2015 297 890

## Description

### Cross-reference to related applications

US 3395697 A (1968) ,,Acoustic reflexometer" discloses a contralateral reflex testing apparatus that detects the acoustic reflex directly as an air pressure variation in the occluded, not stimulated ear canal, without any probe tone.

US3757769 A (1973) "Acoustic admittance testing apparatus" discloses an analog admittance test apparatus which can also detect the acoustic reflex. It is based on a single probe tone of a not specified frequency and implements quadrature detection.

US 3949735 A (1976) "Method and apparatus for an ipsilateral reflex test" discloses an apparatus for testing the ipsilateral acoustic reflex. It mixes both a 220 Hz probe tone and 200 ms pulses of a stimulus to one probe speaker. The stimulus timing gates a comparator circuit which compares the filtered microphone signal between stimulated and non-stimulated phase. This appears to be the first ipsilateral acoustic reflex testing apparatus.

US 4201225 A (1980) "Method and apparatus for measuring stimulated acoustic reflex latency time" discloses a method to measure the latency of the acoustic reflex with a 220 Hz probe tone.

US 4966160 A (1990) "Acoustic admittance measuring apparatus with wide dynamic range and logarithmic output" discloses an apparatus for testing the ipsilateral acoustic reflex with a settable, single probe tone in the range of 150 Hz to 2.5 kHz and an exponential detection circuit to allow a wide dynamic range. The inventor states that existing instruments only allow probe tones of 226 and 678 Hz exclusively.

US 6295467 B1 (2001) "Method and device for detecting a reflex of the human stapedius muscle" discloses a method to use the stimulus signal itself to detect the ipsilateral reflex, instead of applying a separate probe tone. Pairs of signal bursts are presented within a short period of time, and the recorded sound of both is compared to detect the acoustic reflex, assuming only the latter of the stimuli is impacted by the reflex, due to its latency.

US 8419655 B2 (2013) "Method and apparatus for aural acoustic immittance measurement" discloses an apparatus for immittance testing that uses FFT to convert the microphone signal to frequency domain. It still uses a monofrequent probe tone.

US 20130303941 A1 (2013) "Method and Apparatus for Evaluating Dynamic Middle Ear Muscle Activity" discloses a method to test the contralateral acoustic reflex with a broad band signal as the probe tone. Because of the nature of the probe signal, ipsilateral testing is not possible. The method aims at getting more information about the dynamics of the middle ear.

US2012302859 A1 (2012), FR2971931 A1 (2012), and US2015297890 A1 (2015) disclose devices and methods for measuring the stapedius reflex. US5885225 A (1999) discloses a device and method for measuring evoked otoacoustic emissions.

### Non-patent references

Freeney M., Keefe D. H. (1999). Acoustic Reflex Detection Using Wide-Band Acoustic Reflectance, Admittance, and Power Measurements. Journal of Speech, Language, and Hearing Research Vol. 42, 1029-1041

Peake WT, Rosowski JJ. (1997). Acoustic properties of the middle ear. In: MJ Crocker (Eds.)Encyclopedia of Acoustics. (pp. 1337-1346) New York: Wiley.

Rosowski J., Wilber L.A. (2015) Acoustic Immittance, Absorbance, and Reflectance in the Human Ear Canal. Semin Hear 2015; 36(01): 11-28

Liu Y-W, Sanford CA, Ellison JC, Fitzpatrick DF, Gorga MP, Keefe DH. (2008) Wideband absorbance tympanometry using pressure sweeps: System development and results on adults with normal hearing. The Journal of the Acoustical Society of America. 2008;124(6):3708-3719. doi:10.1121/1.3001712.

Statement regarding federally sponsored research or development

No federally sponsored research or development is involved in the invention.

### Background of the invention

The disclosed invention belongs to the field of audiometry. Audiometry, as a branch of audiology, is the science of measuring hearing. A subsection of Audiometry deals with objective methods, which do not need the subject, typically a mammal, e.g. a human, under test to cooperate. One of these methods uses the so-called acoustic reflex for objective measurements.

The acoustic reflex is a middle ear muscle activity, triggered by an acoustic stimulus, which results in a tension to either the ear drum or the ossicles. The effect of this muscle activity is to reduce the mobility of the ear drum. This reduced mobility can, among other methods, be detected acoustically. Sine no cooperative subject feedback is needed, this method therefore belongs to the class of objective hearing tests.

A probe for testing the acoustic reflex contains at least one speaker and one microphone. Often, means for applying a static air pressure are also provided. This can be used to compensate middle ear internal pressure in case of tube dysfunction to optimize the general mobility of the ear drum. Therefore, the acoustic reflex test is often included into tympanometers which measure the ear drum mobility as a function of static air pressure. These instruments contain a pump to apply static air pressure to the outer ear. Tympanometry measures the ear drum mobility as a function of static air pressure (relative to ambient pressure), with the best mobility normally occurring at zero relative pressure.

The ear drum mobility is usually described by its "compliance". Since the ear probe roughly emits a constant volume flow q, independent from the acoustic load, the sound pressure that is measured by the microphone acts inverse to the compliance. A higher compliance, indicating higher mobility, would reduce the recorded probe tone sound level. The instrument is typically calibrated to display the compliance as an equivalent acoustic air volume or equivalent acoustic volume in cm³.

The procedure to detect the acoustic reflex involves the presentation of a low level probe tone, typically at 226 Hz, that is recorded back by the probe's microphone. The reflex is then triggered by an additional acoustic stimulus, either presented at the same ear (ipsilateral) or the opposite ear (contralateral). For ipsilateral reflex tests, a second speaker is often build into the probe to decouple the stimulus from the ongoing probe tone. The probe tone amplitude and/or phase component in the recorded signal changes when the ear drum is stiffened by the acoustic reflex. For a low frequency probe tone, such as 226 Hz, the amplitude of the recorded pilot tone signal would usually rise, indicating a lower compliance. For recording, the microphone signal is usually fed through a narrow band filter that is tuned to the probe tone frequency, to remove the acoustic stimulus (if ipsi-lateral) and noise. This filtering can be implemented analog or digitally. The narrow band filter makes the method relatively robust against external noise. The filter must be wide enough to follow admittance changes fast enough, and narrow enough to provide sufficient noise filtering.

However, certain artifacts, such as movement or swallowing, create changes in the recorded probe tone which can be very similar to those caused by the acoustic reflex. These artifacts basically change the residual volume of the ear canal which results in a probe tone change of the same order as a reflex. The system cannot separate between reflex and artifact; it can only assume there is no reflex when no stimulus was presented. Therefore, suppression or detection of this type of artifacts in reflex testing is desirable to make the test more robust and reliable. This is where the present disclosure steps in.

### Brief summary of the invention

It is an object of the invention to improve or to reduce one or more of the downsides of known methods and devices for measuring acoustic reflexes of the ear of a mammal, e.g. a human being.

Aspects of the invention relate to the devices and methods of the independent claims.

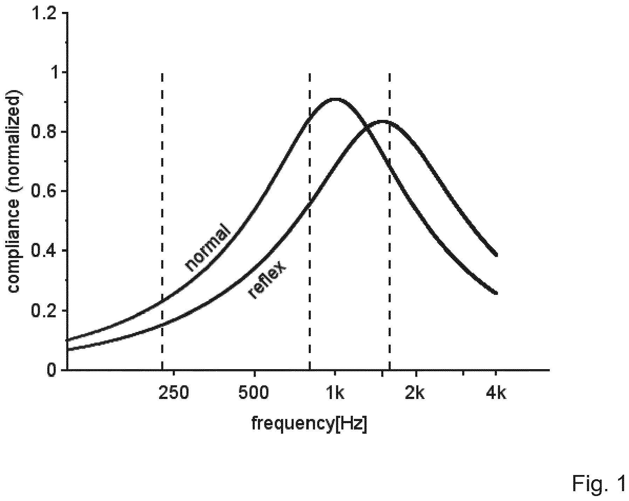

The normal ear drum has its resonance at about 1 kHz. This means, at 1 kHz its mobility is highest. During the acoustic reflex, the middle ear gets stiffened, which results in the resonance to move upwards in frequency. Fig. 1 illustrates the general effect. Obviously, the usual probe tone signal frequency of 226 Hz is clearly below the resonance, and the ear drum would appear stiffer when the reflex is active. A probe frequency not too far above the resonance, however, can show an inverted behavior, since the middle ear resonance moves closer to this probe frequency during the reflex.

Real measured data which supports Fig. 1 can be found in Peake WT, Rosowski JJ. (1997), pp 1343.

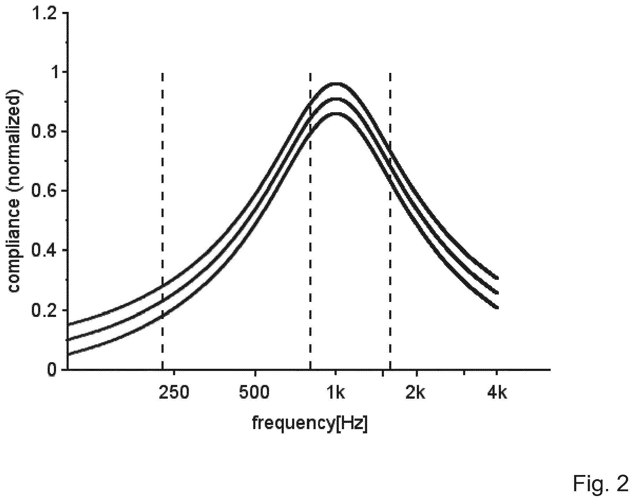

The most typical artifact, caused by mechanical movement, basically modulates the residual ear canal volume (the volume from probe tip to the ear drum). The total compliance will change with this volume change, but in contrast to the reflex, the change will be more similar for various probe frequencies. The effect is illustrated in Fig. 2.

The invention as disclosed here typically makes use of this effect by applying at least two different probe tones simultaneously to test the reflex. In a typical configuration, at least one tone would be placed below the normal middle ear resonance frequency, and at least one above. A reflex would basically cause a decrease of compliance at the lower probe frequency, and an increase at the higher probe frequency. Mechanical artifacts, in contrast to this, would alter both compliance values to the same direction (both up or both down). Therefore, reflex and artifacts can be distinguished from each other, allowing artifact detection and / or suppression.

The principle can both be applied to visual interpretation of the reflex and automated detection. In a visual interpretation setup, curves from different probe tones can be plotted separately, allowing the operator to discriminate the reflex from artifacts. Additionally or alternatively, sum and / or difference traces can be provided where difference traces would contain more reflex activity while sum traces would contain more artifacts.

In an automated setup, the system, e.g. typical methods and apparatuses according to embodiments of the invention, can evaluate all traces and, by fixed or adaptive rules, judge between reflex and artifacts. An adaptive solution would use no-stimulus periods in the measurement session to evaluate the correlation of different probe tone feedback signals. No-Stimulus periods would just contain artifacts, so that the system, e.g. typical methods and apparatuses according to embodiments of the invention, can "learn" how artifacts impact the admittances.

The method can as well be applied to record acoustic reflexes from external stimuli, such as cochlea implants.

### Brief description of the drawings

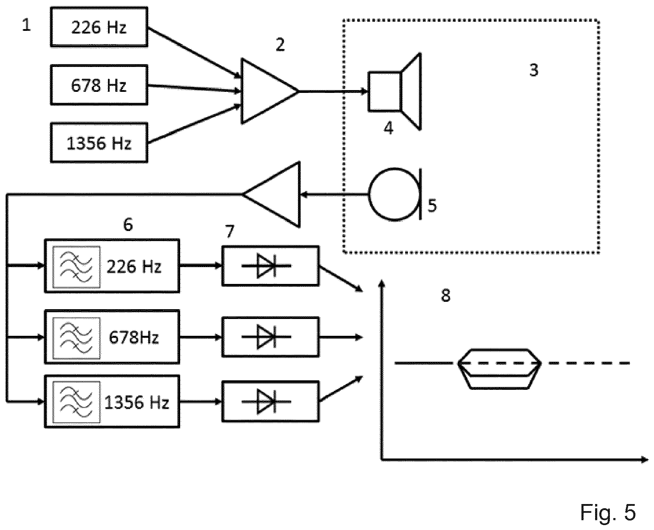

Fig. 1 illustrates the admittance change with the acoustic reflex: The resonance frequency, which corresponds to a maximum in admittance, moves to higher frequencies. The change in admittance that can be recorded at 3 different frequencies differs. Real, measured data can be found in Peake WT, Rosowski JJ. (1997), pp 1343, Fig 5 a.
Fig. 2 illustrates the admittance change with probe movement (typical artifact). The residual volume is modulated, resulting in a similar change in admittance for most frequencies.

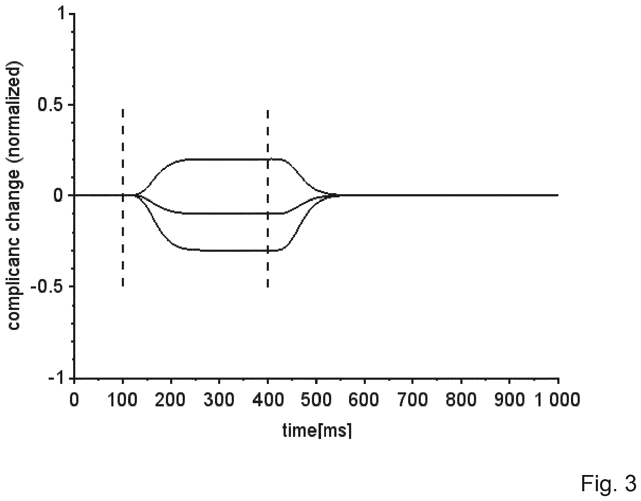

Fig. 3 illustrates a 3-channel recording of a reflex. Since the three traces correspond to different probe frequencies, one of which is above the middle ear resonance frequency, they respond differently to the reflex.

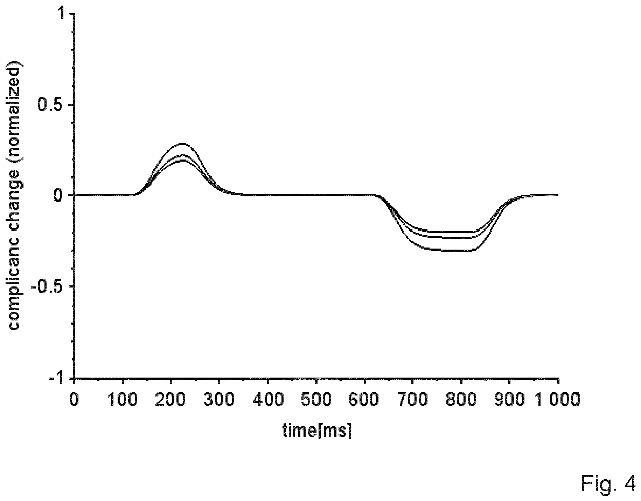

Fig. 4 illustrates a 3-channel recording of volume-changing artifacts. Since a change of the residual volume changes the measured compliance of all frequencies to the same direction, the response of all three channels is to the same sign. Both directions (volume smaller, volume bigger) can occur.
Fig. 5 illustrates a simplified principle schematic of the method. Three different probe frequencies are generated 1, amplified 2 and presented to the ear probe's 3 speaker 4. The ear probe's microphone 5 records back the signal which is amplified and separated by band pass filters, e.g. narrow band filters 6, which are tuned to the probe frequencies. The amplitude of the component frequencies is measured 7, offset removed and presented 8. Means to trigger the reflex is not shown for simplicity. It can be acoustically on either ear or electrical (e.g. via a cochlea implant).

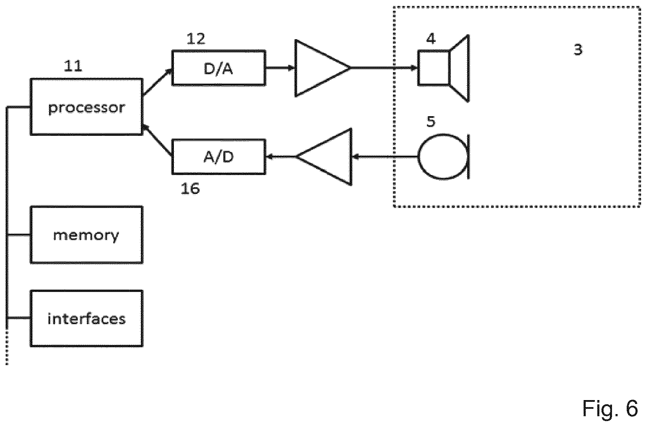

Fig. 6 illustrates a more realistic implementation of the invention, where all signal processing is implemented digitally. A processor 11 is connected to a D/A converter 12 and a A/D converter 16 which provide probe tone and stimulus output and recording of the microphone signal. The processor can be connected to various interfaces 17 which can be a direct user interface, such as display, keyboard, touch-screen, or a connection to a computer for operation. Again, means to trigger the reflex is not shown.

### Detailed description of embodiments of the invention

The human middle ear and at least some of the middle ears of mammals in general transform the external sound field that enters the ear canal to a sound in the inner ear. Since the inner ear is filled with a fluid, its acoustic impedance is different, basically towards higher sound pressure and lower medium velocity. The middle ear handles this by connecting a relatively big ear drum membrane to a small stapes footplate, acting as a piston into the inner ear. The middle ear efficiency peaks at a frequency of around 1 kHz. This frequency corresponds to the main resonance of the middle ear, for example, where mobility is highest. The mobility is usually described as compliance, which is in fact the imaginary part of the so-called acoustic admittance. Acoustic impedance is defined as p/q, acoustic admittance as q/p, where p is the sound pressure and q is the sound volume flow (volume / time).

A muscle, called musculus stapedius, is connected to the stapes bone and, if activated, drags the stapes bone side wards. This temporarily reduces the mobility of the stapes bone and in turn the ear drum. However, since the muscle stiffens the overall middle ear mechanics, it also moves its resonance frequency up.

The musculus stapedius is activated when loud sound is received by either ear, and is thought to be a protective mechanism. Other purposes are under discussion. Since the muscle acts non-voluntary, its activation is called stapedius reflex or acoustic reflex. The sound level that is needed to excite the stapedius reflex is called acoustic reflex threshold, herein also referred to as the threshold of the acoustic reflex. According to some embodiments, which can be combined with other embodiments described herein, the threshold of the acoustic reflex can be at 50 dB HL or above, such as 70 dB HL or above.

In audiometry, the acoustic reflex can be used for objective measurements, since the subject under test does not have to voluntary respond to the examiner. This makes this test possible in sleeping subjects, e.g. young children, or during surgery. It can also be used in context with implantable hearing devices, such as cochlea implants, which stimulate the inner ear electrically.

The standard method to record the stapedius reflex is to apply a moderate level, low frequency tone of typically 226 Hz to the ear canal via a probe. The probe tone level needs to be well below the reflex threshold. The probe is inserted into the ear canal. The probe also contains a microphone that records back the 226 Hz signal. A stimulus is then applied to the same ear ("ipsilateral") or the opposite ear ("contralateral"). A separate speaker may be provided for this. The 226 Hz component in the microphone signal will change slightly in level and/or phase while the stapedius reflex is present and temporarily stiffens the ear drum. According to embodiments of the present disclosure, means for presentation of a stimulus can be a loudspeaker, such as the loudspeaker for the probe tone or a separate lout speaker, or can be an electrode configure to provide an electrical stimulus.

Usually, the instruments that record the acoustic reflex are combined in so-called tympanometers. Tympanometry measures the mobility of the ear drum as a function of external static air pressure, and is a diagnostic method for the middle ear. The method to detect the change in mobility via a probe tone is closely related to acoustic reflex test, which is why the methods are often combined. If the tympanometry pump is available in the reflex system, applying a static pressure to a value where the ear drum mobility is maximal, can improve reflex testing reliability. Therefore, before reflex testing, the tympanometry is often executed to determine this pressure of maximum compliance.

In tympanometry, alternate frequencies are often provided which can be used instead of the standard 226 Hz, such as 1 kHz. An alternate approach ("wide band tympanometry", Liu et.al 2008) uses broad band clicks to calculate the admittance over a broad frequency band as a function of static pressure. A similar approach is proposed by Freeney M., Keefe D. H. (1999). However, the repetition rate of the click is somewhat limited, its sound level needs to be quite high and some averaging is needed to achieve a sufficient signal-to-noise-ratio (SNR). This means the detection signal is at risk to evoke the reflex by itself.

The standard frequency of 226 Hz in tympanometry and reflex testing is historically selected because of a relation between acoustic admittance and effective acoustic volume: At 226 Hz, the acoustic volume in cm³ and the acoustic compliance are numerically equivalent. The frequency of 226 Hz is below the middle ear resonance. The middle ear system acts as a mass-spring-system. At frequencies below the resonance, it mainly acts as a spring, and the reflex will stiffen this spring.

Since the stapedius reflex will shift the middle ear resonance towards higher frequencies, the compliance at a higher probe frequency, where the middle ear mainly acts as a mass, can also rise during the reflex. Therefore, using a probe tone above the middle ear resonance, results in an inverted behavior of the compliance change during the reflex. This is illustrated in Fig. 1.

Since the change in the microphone signal due to the acoustic reflex is small, the measurement can easily be disturbed by artifacts. One obvious artifact is external noise that can interfere with the probe tone recording. This can be handled quite well by using a narrow band filter for the microphone signal. In state-of-the-art implementations, this filtering is implemented digitally. This narrow-band recording allows very moderate probe tone levels, in contrast to wide band approaches.

Another type of artifact includes any movement of the probe position within the ear canal. Such a movement will change the residual volume between probe and ear drum. Since the residual volume contributes to the overall compliance that the probe measures, a change can easily be mistaken as a reflex. A similar artifact occurs if the subject under test swallows or yawns.

As a consequence, an experienced examiner or somebody who is aware of the artifacts may tend to increase stimulus levels well above the threshold until a clear reflex is recorded. The threshold would then be overestimated. It may also be necessary to repeat testing specific stimuli until artifact-free recordings are achieved, thus prolonging total examination time.

An artifact as described above will modulate the residual volume. The compliance change during the artifact can therefore be expected to be similar for various probe frequencies. In contrast to this, the acoustic reflex, since it shifts the middle ear resonance, will act differently for different probe frequencies.

The present invention typically uses this different behavior for artifact suppression. Typical embodiments of the invention present more than one probe tone frequency simultaneously, and record all of them back. The different probe frequencies can be separated via dedicated narrow band filters from the common microphone signal, so that more than one compliance values can be derived simultaneously at any time. The overall loudness of the tones can still be kept moderate enough to be safe against triggering the reflex.

According to some embodiments, distinct frequencies may be used for the one or more probe tone. According to implementations with a broad band noise and Fourier transformation of the measurement, the overall volume can be reduced since a good signal to noise ration can be provided at reduced volume. For example, the signal to noise ration can be improved by filters having a bandwidth of 30 Hz or below, e.g. about 10 Hz.

If, for example, frequencies 226 Hz, 678 Hz, 1356 Hz (1, 3, 7 times 226 Hz) are used, compliance at 226 Hz would drop, compliance at 678 Hz would not change much, and compliance at 1356 Hz would rise during the acoustic reflex. In contrast to this, the measured compliance at all frequencies would rise if the probe is moved outwards or drop if it is moved inwards the ear canal, due to a mere residual volume change. Even if, the compliance change does not change the sign for two different frequencies, the compliance change will have a different increase or decrease, respectively, allowing to separate the measurement signal from an artifact.

The test system or typical embodiments can therefore separate artifacts and reflex in the response data. This separation can either be done by the examiner, who observes, for example, three traces instead of just one, knowing how reflex response and artifact response look like. Typically, the data is evaluated automatically, and a derived, artifact-free reflex signal is shown to the examiner. Such evaluation may contain calculation of differences of compliance traces, correlation methods, etc. Typical embodiments may also make use of the phases of the recorded signals. A preferred implementation weights the recorded traces with factors (some of which are negative), which are selected to result in an artifact-free reflex response trace.

An apparatus which implements typical methods as described herein would typically contain a processor and digital-analog and analog-digital conversion. All signal generation and recording is typically implemented digitally. The filters would typically be implemented using quadrature detection, which allows phase-locked recording. In such an implementation, the number of probe tones that are used simultaneously is only limited by the overall sound level they produce, which needs to be below the reflex threshold, and the processing power of the digital system. A typical implementation would use at least three probe tones.

The configuration of the probe tones (number and frequencies) can be configurable, to, for example, optimally handle middle ears of different age groups or middle ear disorders. Said factors for deriving an artifact-free signal could also be adjustable or even automatically "learned" by the system during no-stimulus phases.

Typically, means for evaluation of recorded probe tones are adapted to evaluate the phase of the recorded tones to separate reflex from artifact response. With typical embodiments a signal generation and/or signal analysis is implemented digitally. Typically, a tympanometric pump is used to allow reflex testing under static pressure. Typical methods and apparatuses automatically select stimulus frequencies and/or levels and automatically find the subject's reflex threshold based on the automatically selected stimulus frequencies and/or levels.

## Claims

1. An apparatus for measuring the acoustic reflex that presents and records at least two probe tones of different frequencies simultaneously in an ear canal of a human, comprising
a) a probe (3) with at least one speaker (4) and at least one microphone (5), adapted to be placed in an ear canal of a human;
b) means for signal generation and presentation via said at least one speaker (4) of at least two probe tones of different frequencies simultaneously;
c) means for recording back said at least two probe tones via said at least one microphone (5) and band pass filters tuned to the probe frequencies; and
d) means for evaluation of said recorded probe tones for amplitudes and / or phases to detect an acoustic reflex and/or artifacts, wherein the means for evaluation, a processor and means for digital-analog conversion and means for analog-digital conversion perform signal generation, recording and evaluation of the method according any of claims 10 to 18.

2. The apparatus according to claim 1 which provides means for presentation of a stimulus, either ipsilateral or contralateral or both, to trigger the acoustic reflex.

3. The apparatus according to claim 1 or 2, that provides an interface for an external trigger to allow detection of the acoustic reflex from external stimulation.

4. The apparatus according to any one of the proceeding claims, with means for displaying the compliance as derived from the recorded probe tones and/or other features of those signals graphically for visual inspection.

5. The apparatus according to any one of the proceeding claims, wherein the means for evaluation are adapted to automatically detect the acoustic reflex by using two or more of the recorded probe tone signals simultaneously.

6. The apparatus according to any one of the proceeding claims, wherein the means for evaluation are adapted to automatically detect or suppress artifacts by using two or more of the recorded probe tone signals.

7. The apparatus according to any one of the proceeding claims, wherein the means for evaluation are adapted to automatically adapt artifact suppression parameters during no-stimulus phases.

8. The apparatus according to any one of the proceeding claims, that includes a tympanometric pump to allow reflex testing under static pressure.

9. The apparatus according to any one of the proceeding claims, wherein the means for evaluation are adapted to automatically select stimulus frequencies and/or levels to automatically find a threshold of the acoustic reflex.

10. A method for measuring the acoustic reflex that presents and records at least two probe tones of different frequencies simultaneously, comprising
placing a probe with at least one speaker in an ear canal of a human;
signal generation and presentation via the at least one speaker (4) of at least two probe tones of different frequencies simultaneously;
recording back said at least two probe tones influenced in amplitudes and/or phases by an ear drum compliance of the ear canal via at least one microphone (5) and band pass filters tuned to the probe frequencies; and
evaluation of said recorded probe tones for amplitudes and / or phases to detect an acoustic reflex and/or artifacts.

11. The method according to claim 10, comprising a presentation of a stimulus, either ipsilateral or contralateral or both, to trigger the acoustic reflex.

12. The method according to claim 10 or 11, providing an external trigger to allow detection of the acoustic reflex from external stimulation.

13. The method according to any one of the claims 10 to 12, comprising displaying the compliance as derived from the recorded probe tones and/or other features of those signals graphically for visual inspection.

14. The method according to any one of the claims 10 to 13, comprising automatically detecting the acoustic reflex by using two or more of the recorded probe tone signals simultaneously.

15. The method according to any one of the claims 10 to 14, comprising automatically detecting or suppressing artifacts by using two or more of the recorded probe tone signals.

16. The method according to any one of the claims 10 to 15, comprising automatically adapting artifact suppression parameters during no-stimulus phases.

17. The method according to any one of the claims 10 to 16, comprising reflex testing under static pressure.

18. The method according to any one of the claims 10 to 17, comprising automatically selecting stimulus frequencies and/or levels to automatically find a threshold of the acoustic reflex.

## Patentansprüche

1. Eine Vorrichtung zur Messung des akustischen Reflexes, der mindestens zwei Sondentöne unterschiedlicher Frequenzen gleichzeitig in einem Gehörgang eines Menschen präsentiert und aufzeichnet, umfassend
a) eine Sonde (3) mit mindestens einem Lautsprecher (4) und mindestens einem Mikrofon (5), die dazu geeignet ist, in einen Gehörgang eines Menschen plaziert zu werden;
b) Mittel zur Signalerzeugung und Präsentation über den mindestens einen Lautsprecher (4) von mindestens zwei Sondentönen mit unterschiedlichen Frequenzen gleichzeitig;
c) Mittel zum Aufzeichnen der mindestens zwei Sondentöne über das mindestens eine Mikrofon (5) und auf die Sondenfrequenzen abgestimmte Bandpassfilter; und
d) Mittel zur Auswertung der aufgezeichneten Sondentöne für Amplituden und/ oder Phasen zur Erkennung eines akustischen Reflexes und/oder von Artefakten, wobei die Mittel zur Auswertung, ein Prozessor und Mittel zur Digital-Analog-Wandlung und Mittel zur Analog-Digital-Wandlung eine Signalerzeugung, Aufzeichnung und Auswertung des Verfahrens nach einem der Ansprüche 10 bis 18 durchführen.

2. Vorrichtung nach Anspruch 1, die Mittel zur Präsentation eines Reizes, entweder ipsilateral oder kontralateral oder beides, zur Auslösung des akustischen Reflexes bereitstellt.

3. Vorrichtung nach Anspruch 1 oder 2, die eine Schnittstelle für einen externen Trigger bereitstellt, um die Erkennung des akustischen Reflexes durch externe Stimulation zu ermöglichen.

4. Vorrichtung nach einem der vorhergehenden Ansprüche, mit Mitteln zur grafischen Darstellung der aus den aufgezeichneten Sondentönen abgeleiteten Comliance und/oder anderer Merkmale dieser Signale zur visuellen Kontrolle.

5. Vorrichtung nach einem der vorhergehenden Ansprüche, wobei die Mittel zur Auswertung geeignet sind, den akustischen Reflex automatisch zu erkennen, indem zwei oder mehr der aufgezeichneten Sondentonsignale gleichzeitig verwendet werden.

6. Vorrichtung nach einem der vorhergehenden Ansprüche, wobei die Mittel zur Auswertung geeignet sind, Artefakte automatisch zu erkennen oder zu unterdrücken, indem zwei oder mehr der aufgezeichneten Sondentonsignale verwendet werden.

7. Vorrichtung nach einem der vorhergehenden Ansprüche, wobei die Mittel zur Auswertung geeignet sind, Artefaktunterdrückungsparameter während reizfreier Phasen automatisch anzupassen.

8. Vorrichtung nach einem der vorhergehenden Ansprüche, die eine tympanometrische Pumpe aufweist, um eine Reflexprüfung unter statischem Druck zu ermöglichen.

9. Vorrichtung nach einem der vorhergehenden Ansprüche, wobei die Mittel zur Auswertung geeignet sind, automatisch Reizfrequenzen und/oder -pegel auszuwählen, um automatisch eine Schwelle des akustischen Reflexes zu finden.

10. Verfahren zur Messung des akustischen Reflexes, bei dem mindestens zwei Sondentöne mit unterschiedlichen Frequenzen gleichzeitig präsentiert und aufgezeichnet werden, umfassend
Platzieren einer Sonde mit mindestens einem Lautsprecher in einen Gehörgang eines Menschen;
Signalerzeugung und Präsentation über den mindestens einen Lautsprecher (4) von mindestens zwei Sondentönen unterschiedlicher Frequenzen gleichzeitig;
Aufzeichnung der mindestens zwei Sondentöne, die durch eine Trommelfellcompliance des Gehörgangs in ihren Amplituden und/oder Phasen beeinflusst werden, über mindestens ein Mikrofon (5) und auf die Sondenfrequenzen abgestimmte Bandpassfilter; und
Auswertung der aufgezeichneten Sondentöne für Amplituden und/ oder Phasen zur Erkennung eines akustischen Reflexes und/oder von Artefakten.

11. Verfahren nach Anspruch 10, umfassend eine Präsentation eines Reizes, entweder ipsilateral oder kontralateral oder beides, um den akustischen Reflex auszulösen.

12. Verfahren nach Anspruch 10 oder 11, wobei ein externer Trigger vorgesehen ist, um die Erkennung des akustischen Reflexes durch externe Stimulation zu ermöglichen.

13. Verfahren nach einem der Ansprüche 10 bis 12, bei dem die aus den aufgezeichneten Sondentönen abgeleitete Compliance und/oder andere Merkmale dieser Signale zur visuellen Kontrolle grafisch dargestellt werden.

14. Verfahren nach einem der Ansprüche 10 bis 13, umfassend das automatische Erkennen des akustischen Reflexes durch gleichzeitige Verwendung von zwei oder mehr der aufgezeichneten Sondentonsignale.

15. Verfahren nach einem der Ansprüche 10 bis 14, umfassend das automatische Erkennen oder Unterdrücken von Artefakten unter Verwendung von zwei oder mehr der aufgezeichneten Sondentonsignale.

16. Verfahren nach einem der Ansprüche 10 bis 15, umfassend das automatische Anpassen von Artefaktunterdrückungsparametern während reizarmer Phasen.

17. Verfahren nach einem der Ansprüche 10 bis 16, umfassend eine Reflexionsprüfung unter statischem Druck.

18. Verfahren nach einem der Ansprüche 10 bis 17, umfassend das automatische Auswählen von Reizfrequenzen und/oder -pegeln, um automatisch eine Schwelle des akustischen Reflexes zu finden.

## Revendications

1. Appareil pour mesurer le réflexe acoustique qui présente et enregistre au moins deux tonalités de sonde de différentes fréquences simultanément dans un conduit auditif d'un être humain, comprenant :
a) une sonde (3) avec au moins un haut-parleur (4) et au moins un microphone (5), adaptée pour être placée dans un conduit auditif d'un être humain ;
b) des moyens pour la génération et la présentation de signal via ledit au moins un haut-parleur (4) d'au moins deux tonalités de sonde de différentes fréquences simultanément ;
c) des moyens pour l'enregistrement desdites au moins deux tonalités de sonde via ledit au moins un microphone (5) et de filtres passe-bande accordés aux fréquences de sonde ; et
d) des moyens pour l'évaluation desdites tonalités de sonde enregistrées pour les amplitudes et/ou les phases pour détecter un réflexe acoustique et/ou des artefacts, dans lequel les moyens pour l'évaluation, un processeur et des moyens pour la conversion numérique-analogique et des moyens pour la conversion analogique-numérique réalisent une génération, un enregistrement et une évaluation de signal du procédé selon l'une quelconque des revendications 10 à 18.

2. Appareil selon la revendication 1, qui fournit des moyens pour la présentation d'un stimulus, ipsilatéral ou contralatéral ou les deux, pour déclencher le réflexe acoustique.

3. Appareil selon la revendication 1 ou 2, qui fournit une interface pour un déclenchement externe pour permettre une détection du réflexe acoustique à partir d'une stimulation externe.

4. Appareil selon l'une quelconque des revendications précédentes, avec des moyens pour afficher la compliance telle que déduite des tonalités de sonde enregistrées et/ou d'autres caractéristiques de ces signaux graphiquement pour un contrôle visuel.

5. Appareil selon l'une quelconque des revendications précédentes, dans lequel les moyens pour l'évaluation sont adaptés pour détecter automatiquement le réflexe acoustique en utilisant simultanément deux ou plus des signaux de tonalité de sonde enregistrés.

6. Appareil selon l'une quelconque des revendications précédentes, dans lequel les moyens pour l'évaluation sont adaptés pour détecter ou supprimer automatiquement des artefacts en utilisant deux ou plus des signaux de tonalité de sonde enregistrés.

7. Appareil selon l'une quelconque des revendications précédentes, dans lequel les moyens pour l'évaluation sont adaptés pour adapter automatiquement des paramètres de suppression d'artefact pendant des phases de non stimulus.

8. Appareil selon l'une quelconque des revendications précédentes, qui inclut une pompe tympanométrique pour permettre un test de réflexe sous pression statique.

9. Appareil selon l'une quelconque des revendications précédentes, dans lequel les moyens pour l'évaluation sont adaptés pour sélectionner automatiquement des fréquences et/ou niveaux de stimulus pour trouver automatiquement un seuil du réflexe acoustique.

10. Procédé pour mesurer le réflexe acoustique qui présente et enregistre au moins deux tonalités de sonde de différentes fréquences simultanément, comprenant les étapes de :
placer une sonde avec au moins un haut-parleur dans un conduit auditif d'un être humain ;
génération et présentation de signal via le au moins un haut-parleur (4) d'au moins deux tonalités de sonde de différentes fréquences simultanément ;
enregistrer lesdites au moins deux tonalités de sonde influencées en amplitudes et/ou phases par une compliance de tympan du canal auditif via au moins un microphone (5) et des filtres passe-bande accordés aux fréquences de sonde ; et
évaluation desdites tonalités de sonde enregistrées pour les amplitudes et/ou les phases pour détecter un réflexe acoustique et/ou des artefacts.

11. Procédé selon la revendication 10, comprenant une présentation d'un stimulus, ipsilatéral ou contralatéral ou les deux, pour déclencher le réflexe acoustique.

12. Procédé selon la revendication 10 ou 11, prévoyant un déclenchement externe pour permettre une détection du réflexe acoustique à partir d'une stimulation externe.

13. Procédé selon l'une quelconque des revendications 10 à 12, comprenant l'étape d'afficher la compliance telle que déduite des tonalités de sonde enregistrées et/ou d'autres caractéristiques de ces signaux graphiquement pour un contrôle visuel.

14. Procédé selon l'une quelconque des revendications 10 à 13, comprenant l'étape de détecter automatiquement le réflexe acoustique en utilisant simultanément deux ou plus des signaux de tonalité de sonde enregistrés.

15. Procédé selon l'une quelconque des revendications 10 à 14, comprenant l'étape de détecter ou supprimer automatiquement des artefacts en utilisant deux ou plus des signaux de tonalité de sonde enregistrés.

16. Procédé selon l'une quelconque des revendications 10 à 15, comprenant l'étape d'adapter automatiquement des paramètres de suppression d'artefact pendant des phases de non stimulus.

17. Procédé selon l'une quelconque des revendications 10 à 16, comprenant un test de réflexe sous pression statique.

18. Procédé selon l'une quelconque des revendications 10 à 17, comprenant l'étape de sélectionner automatiquement des fréquences et/ou niveaux de stimulus pour trouver automatiquement un seuil du réflexe acoustique.
